# EUROPEAN PATENT APPLICATION

(11) **EP 3 834 741 A1**
(43) Date of publication of application: **16.06.2021**
(21) Application number: 20212940.9
(22) Date of filing: 10.12.2020
(51) Int. Cl.: A61B 17/064, A61B 17/068, H01M 50/574, H01R 13/713

(54) **SYSTEM AND METHOD FOR TEMPORARILY AND PERMANENTLY DISABLING ELECTRONICS IN A DISPOSABLE SURGICAL TOOL**

(30) Priority: 10.12.2019 US 201962945951 P; 07.10.2020 US 202017064714
(71) Applicant: Covidien LP, Mansfield, MA 02048 (US)
(72) Inventor: FISCHVOGT, Gregory, Reno, Nevada 89521 (US); MORRIS, Michael D., Thornton, Colorado 80241 (US)
(74) Representative: Maschio, Antonio

(57) **Abstract**

A safety cut-off circuit for a surgical instrument includes a liquid detection circuit coupled in parallel to a fuse and a voltage regulator. Power supplied to the voltage regulator is cut-off when liquid comes into contact with the liquid detection circuit.

## Description

### CROSS REFERENCE TO RELATED APPLICATION

The present application claims the benefit of and priority to U.S. Provisional Application Serial No. 62/945,951, filed on December 10, 2019, the entire contents of which are incorporated herein by reference.

### FIELD

The disclosure relates to surgical instruments and, more particularly, to a safety cut-off circuit and a powered surgical tack applier instrument including a safety cut-off circuit.

### BACKGROUND

Various surgical procedures require instruments capable of applying fasteners to tissue to form tissue connections or to secure objects to tissue. For example, during hernia repair it is often desirable to fasten a mesh to tissue. In certain hernias, such as direct or indirect inguinal hernias, a part of the intestine protrudes through a defect in the abdominal wall to form a hernial sac. The defect may be repaired using an open surgery procedure in which a relatively large incision is made and the hernia is closed outside the abdominal wall by suturing. The mesh is attached with sutures over the opening in the abdominal wall to provide reinforcement. However, this may also be accomplished through the use of minimally invasive surgical fasteners such as, e.g., surgical tacks.

Following the surgical procedure, some surgical instruments may be reprocessed for reuse, while others are disposable.

### SUMMARY

The disclosure relates to surgical instruments and, more particularly, to a safety cut-off circuit and a powered surgical tack applier instrument including a safety cut-off circuit.

In accordance with an aspect, a safety cut-off circuit for a surgical instrument includes a positive terminal and a negative terminal, the negative terminal being grounded, a fuse coupled in series to the positive terminal of the power supply, a liquid detection circuit coupled in parallel to the fuse and the negative terminal of the power supply, and a voltage regulator operably coupled to the liquid detection circuit and the positive terminal of the power supply via the fuse. Power supplied to the voltage regulator is cut-off when liquid comes into contact with the liquid detection circuit.

The fuse is configured to blow when liquid contacts the liquid detection circuit. In an aspect, the fuse includes an amperage rating greater than an amperage rating required to operate the safety cut-off circuit.

In an aspect, the liquid detection circuit is coupled to the fuse via a first trace and to the voltage regulator via a second trace and the first trace is of a lower gauge relative to the second trace.

In an aspect, the liquid detection circuit includes water detection traces. In an aspect, the liquid detection circuit includes an interlaced comb structure.

In an aspect, the safety cut-off circuit includes a transistor coupled in parallel to the safety cut-off circuit and configured to be selectively triggered to create a short circuit and blow the fuse. An amperage capacity of the transistor may be higher than an amperage capacity of the fuse. In an aspect, the transistor includes a logic pin coupled to a microcontroller for selectively triggering the transistor to create the short circuit and blow the fuse. The transistor may be triggered to create the short circuit and blow the fuse when at least one of an end of useable life is detected, liquid is detected elsewhere in the surgical instrument remote from the liquid detection circuit, or erroneous behavior or signals are detected from at least one other electrical component of the surgical instrument. The at least one other electrical component may include a motor or a power source.

In an aspect, the safety cut-off circuit includes a resettable fuse coupled in series to the fuse, wherein an amperage rating of the resettable fuse is less than an amperage rating of the fuse. The safety cut-off circuit may further include a first transistor and a second transistor, wherein an amperage capacity of the first transistor is greater than an amperage capacity of the fuse and an amperage capacity of the second transistor is greater than an amperage capacity of the resettable fuse. In an aspect, the second transistor includes a logic pin coupled to a microcontroller for selectively triggering the second transistor to create a short circuit and blow the resettable fuse.

In another aspect of the disclosure, a safety cut-off circuit for a surgical instrument includes a power supply including a positive terminal and a negative terminal, the negative terminal being grounded, a fuse coupled in series to the positive terminal of the power supply, at least one of a liquid detection circuit or a transistor coupled in parallel to the fuse and the negative terminal of the power supply, a voltage regulator operably coupled to at least one of the liquid detection circuit or the transistor and the positive terminal of the power supply via the fuse. Power supplied to the voltage regulator is cut-off when at least one of liquid comes into contact with the liquid detection circuit or the transistor is caused to short circuit the safety cut-off circuit and blow the fuse.

In yet another aspect of the disclosure, a powered surgical instrument includes a handle assembly, an articulation lever assembly, an elongate member, and a safety cut-off circuit operably coupled to at least one of the handle assembly, the articulation lever assembly, or the elongate member. The handle assembly includes an actuation assembly including a motor, an actuation rod having a first end operatively coupled to an output shaft of the motor for concomitant rotation therewith, and an actuation switch configured to actuate the motor. The articulation lever assembly includes an articulation rod and an articulation lever operatively coupled with the articulation rod. The safety cut-off circuit includes a power supply including a positive terminal and a negative terminal, the negative terminal being grounded, a fuse coupled in series to the positive terminal of the power supply, a liquid detection circuit coupled in parallel to the fuse and the negative terminal of the power supply, and a voltage regulator operably coupled to the liquid detection circuit and the positive terminal of the power supply via the fuse. Power supplied to the voltage regulator is cut-off when liquid comes into contact with the liquid detection circuit.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various aspects of the disclosure are described hereinbelow with reference to the drawings, which are incorporated and constitute a part of this specification, wherein:
FIG. 1 is a perspective view of a handle assembly of a powered surgical tack applier in accordance with an aspect of the disclosure;
FIG. 2 is a partial perspective view of an elongate member of the powered surgical tack applier;
FIG. 3 is a partial perspective view of a loading unit of the surgical tack applier of FIG. 1, illustrating a coil separated from an inner tube;
FIG. 4 is a longitudinal, cross-sectional view of a distal end of the powered surgical tack applier, illustrating implanting of a surgical tack into underlying tissue through a surgical mesh;
FIG. 5 is a perspective view of a surgical mesh for use with the powered surgical tack applier of FIG. 1, illustrating anchoring the surgical mesh to underlying tissue with a plurality of surgical tacks;
FIG. 6 is a side view of the handle assembly of FIG. 1 with a half of a housing removed;
FIG. 7 is an exploded perspective view of the handle assembly of FIG. 1 with parts separated;
FIG. 8 is a partial side view of the handle assembly of FIG. 1;
FIG. 9 is a partial side view of the handle assembly of FIG. 1 with a portion of the housing removed;
FIG. 10 is a partial perspective view of the handle assembly of FIG. 1, illustrating an actuation assembly;
FIG. 11 is a perspective view of a handle assembly for use with a powered surgical tack applier in accordance with another aspect of the disclosure;
FIG. 12 is a perspective view of the handle assembly of FIG. 11 with a half of the housing removed;
FIG. 13 is a side view of the handle assembly of FIG. 11;
FIG. 14 is a circuit diagram of a safety cut-off circuit for a powered surgical instrument in accordance with an aspect of the disclosure;
FIG. 15 is a circuit diagram of a safety cut-off circuit for a powered surgical instrument in accordance with an aspect of the disclosure; and
FIG. 16 is a circuit diagram of a safety cut-off circuit for a powered surgical instrument in accordance with an aspect of the disclosure.

### DETAILED DESCRIPTION

Aspects of the disclosed surgical instrument and its components are described in detail with reference to the drawings, in which like reference numerals designate identical or corresponding elements in each of the several views. As used herein, the term "distal," as is conventional, will refer to that portion of the instrument, apparatus, device, or component thereof which is farther from the user, while the term "proximal" will refer to that portion of the instrument, apparatus, device, or component thereof which is closer to the user. In the following description, well-known functions or constructions are not described in detail to avoid obscuring the disclosure in unnecessary detail.

In electrically powered laparoscopic surgical devices, there often is a need to permanently disable electronic components due to an event occurring during a product's life. Manufacturers may choose to permanently disable electronics as a means of mitigating patient and surgeon hazard in the event of liquid ingress or to ensure that devices are not used beyond their known safe useful life. In both instances disabling the electronic components would allow the device to "fail safe."

Following the surgical procedure, some surgical instruments may be reprocessed for reuse, while others are disposed of. A need exists for disabling surgical instruments that are to be disposed of in order to inhibit their reuse beyond their useful life and for ensuring the safety of the clinician and patient in the event of a faulty condition.

This disclosure provides electronic solutions to address the above-noted concerns. Multiple aspects using either liquid detection circuits (e.g., interlaced comb circuits) or one or more transistors to create short circuits combined with board-mounted fuses are described. The use of passive components that fail due to liquid ingress or other fault conditions ensures that if the microcontroller logic of the surgical instrument or signals become compromised, the device will still safely be able to turn itself off.

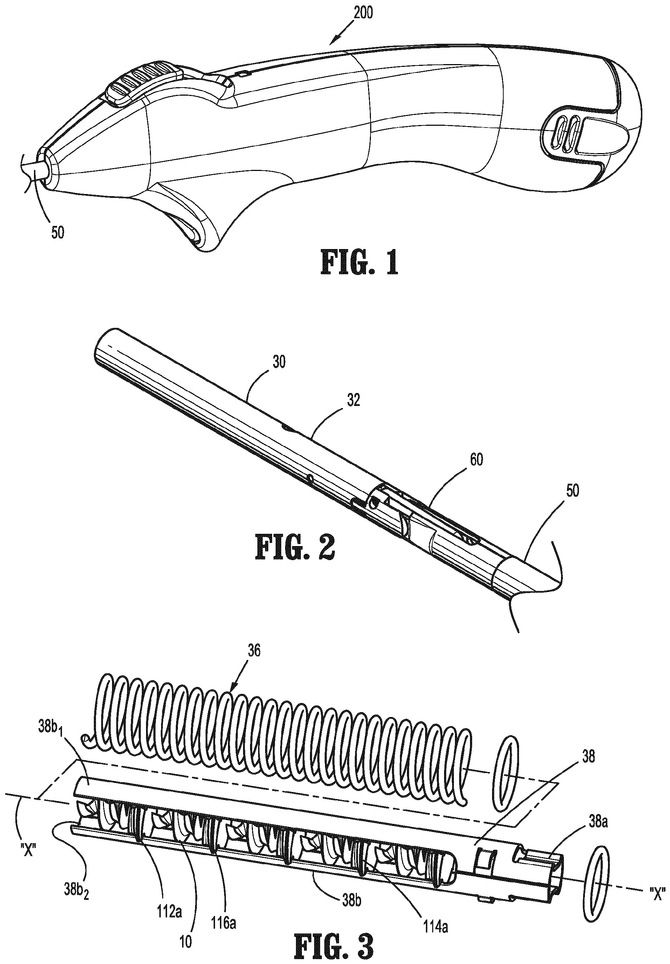

With reference to FIGS. 1-4, a handle assembly for use with a surgical tack applier for applying a surgical tack 10 suitable for insertion through a surgical mesh "M" and tissue "T" is shown generally as a handle assembly 200. The surgical tack applier generally includes the handle assembly 200, an elongate member 50 having an articulation portion 60, and a loading unit 30 selectably connectable to a distal end of the elongate member 50. The loading unit 30 is electro-mechanically coupled to the handle assembly 200 and supports a plurality of surgical tacks 10.

The loading unit 30 includes an outer tube 32 defining a lumen (not shown), a spiral or coil 36 fixedly disposed within the outer tube 32, and an inner tube 38 rotatably disposed within the coil 36. The inner tube 38 defines a lumen therethrough, and includes a first portion 38a and a splined second portion 38b. The second portion 38b of the inner tube 38 is slotted, defining a pair of tines 38bi and a pair of channels 38b₂. The second portion 38b of the inner tube 38 is configured to support the plurality of surgical tacks 10 within the inner tube 38. In particular, the surgical tacks 10 are loaded into the loading unit 30 such that the pair of opposing threaded sections 112a of the surgical tacks 10 extend through respective channels 38b₂ of the second portion 38b of the inner tube 38 and are slidably disposed within the groove of the coil 36, and the pair of tines 38bi of the second portion 38b of the inner tube 38 are disposed within the pair of slotted sections 116a of the surgical tack 10. In use, as the inner tube 38 is rotated about a longitudinal axis "X-X" thereof, relative to the coil 36, the pair of tines 38bi of the inner tube 38 transmits the rotation to the surgical tacks 10 and advance the surgical tacks 10 distally as the head threads 114a of the surgical tacks 10 engage with the coil 36.

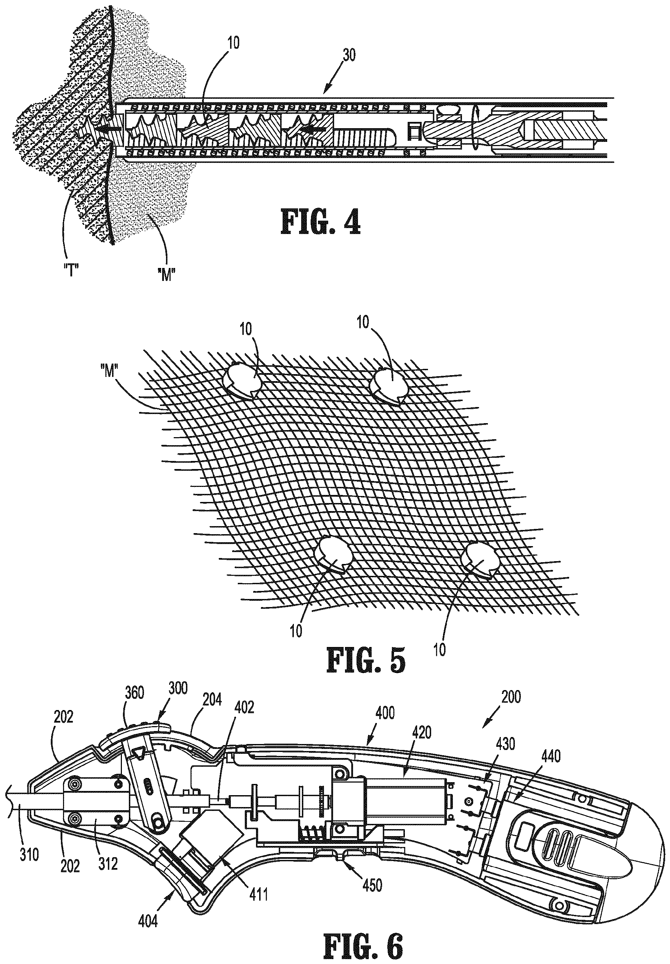

With particular respect to FIG. 2, the surgical tack applier includes an articulation portion 60 operatively coupled with an articulation lever assembly 300 (FIG. 6) supported in the handle assembly 200. The articulation portion 60 may include a drive assembly (not shown) having a slidable tube and an articulation arm pivotally coupled to the slidable tube. The articulation lever assembly 300 is coupled to the slidable tube so that when the articulation lever assembly 300 is actuated the slidable tube is displaced through the elongated member 50. Longitudinal translation of the slidable tube moves the articulation arm to enable the loading unit 30 to articulate relative to the longitudinal axis "X-X" (FIG. 3).

With reference now to FIG. 6, the handle assembly 200 includes a housing 202, an articulation lever assembly 300 configured to articulate the articulation portion 60 (FIG. 2) of the elongate member 50, an actuation assembly 400 configured to eject the surgical tack 10 out of the loading unit 30 of the elongate member 50, and a battery pack 440 removably attached to the housing 202. The housing 202 includes an ergonomic structure providing comfort, ease of use, and intuitiveness such that when the housing 202 is gripped by a clinician, e.g., a thumb, may be positioned to slide the articulation lever assembly 300 and, e.g., an index finger, may be positioned to trigger an actuation switch 404 of the actuation assembly 400. Actuation of the actuation assembly 400 ejects a surgical tack 10 (FIG. 4) out of the loading unit 30 through mesh "M" (FIG. 4) and into body tissue "T".

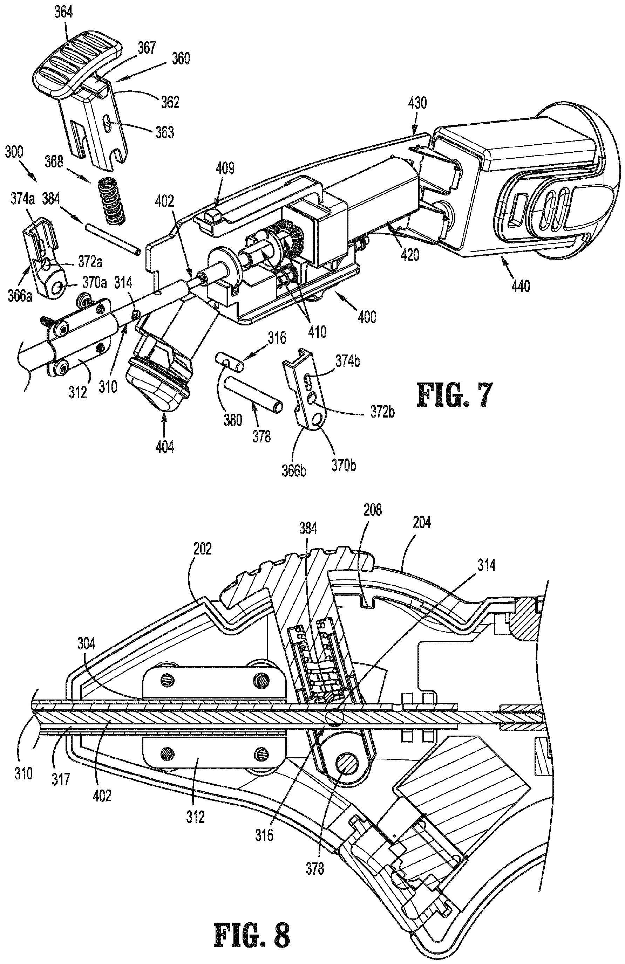

With reference to FIGS. 6 and 7, the articulation lever assembly 300 includes an articulation rod 310 and articulation lever 360 operatively coupled with the articulation rod 310. The articulation rod 310 is operatively coupled with the articulation portion 60 (FIG. 2) of the elongate member 50 of the surgical tack applier. The articulation rod 310 is slidably supported on the housing 202 of the handle assembly 200 by a mounting plate 312 defining a channel 304 (FIG. 8) configured to enable axial displacement of the articulation rod 310 therethrough, which, causes articulation of the articulation portion 60 (FIG. 2) based on the axial position of the articulation rod 310. In particular, the articulation rod 310 has an annular structure defining a channel 317 (FIG. 8) dimensioned to receive the actuation rod 402 of the actuation assembly 400 therein. The articulation rod 310 further defines a transverse bore 314 dimensioned to receive an articulation drive pin 316 coupled with the articulation lever 360.

With continued reference to FIGS. 6 and 7, the articulation lever 360 includes a housing portion 362 and an engaging portion 364 slidably engaging an engaging surface 204 of the housing 202. The engaging surface 204 has an arcuate profile enabling the engaging portion 364 to travel in, e.g., an arc. The housing portion 362 is disposed within the housing 202 and is dimensioned to receive articulation pivot arms 366a, 366b mated together to receive a biasing member 368 therebetween. Each articulation pivot arm 366a, 366b defines a first bore 370a, 370b, a second bore 372a, 372b, and a slot 374a, 374b. The first bores 370a, 370b are dimensioned to receive an articulation pivot pin 378 (FIG. 8) pivotably coupling the articulation pivot arms 366a, 366b to the housing 202. The second bores 372a, 372b are dimensioned to receive the articulation drive pin 316 extending through the transverse bore 314 of the articulation rod 310. Under such a configuration, when the articulation pivot arms 366a, 366b are pivoted about the articulation pivot pin 378, the articulation drive pin 316 causes axial displacement of the articulation rod 310. The articulation drive pin 316 defines a transverse bore 380 dimensioned to receive the actuation rod 402 of the actuation assembly 400 therethrough. The slots 374a, 374b of the articulation pivot arms 366a, 366b are dimensioned to cammingly receive a cam pin 384 biased away from the articulation pivot pin 378 by a biasing member 368 interposed between the articulation pivot arms 366a, 366b.

With reference now to FIGS. 7 and 8, the housing portion 362 of the articulation lever 360 is dimensioned to receive the mated articulation pivot arms 366a, 366b. The housing portion 362 defines a slot 363 dimensioned to cammingly receive the cam pin 384 which is cammingly slidable in the slots 374a, 374b of the articulation pivot arms 366a, 366b. In addition, the housing portion 362 includes a tooth 367 configured to engage a detent portion 208 of the housing 202 to inhibit movement of the articulation lever 360 relative to the housing 202, thereby locking an axial position of the articulation rod 310, which, in turn, locks the orientation of the articulation portion 60 (FIG. 2) of the surgical tack applier. Under such a configuration, the articulation lever 360 is biased away from the articulation pivot pin 378 such that the tooth 367 of the housing portion 362 engages the detent portion 208. When the engaging portion 364 of the articulation lever 360 is depressed towards the housing 202, the tooth 367 is moved away from the detent portion 208 enabling the clinician to slidably move the engaging portion 364 on the engaging surface 204 (FIG. 6) of the housing 202, thereby enabling articulation of the articulation portion 60 of the surgical tack applier to a desired orientation.

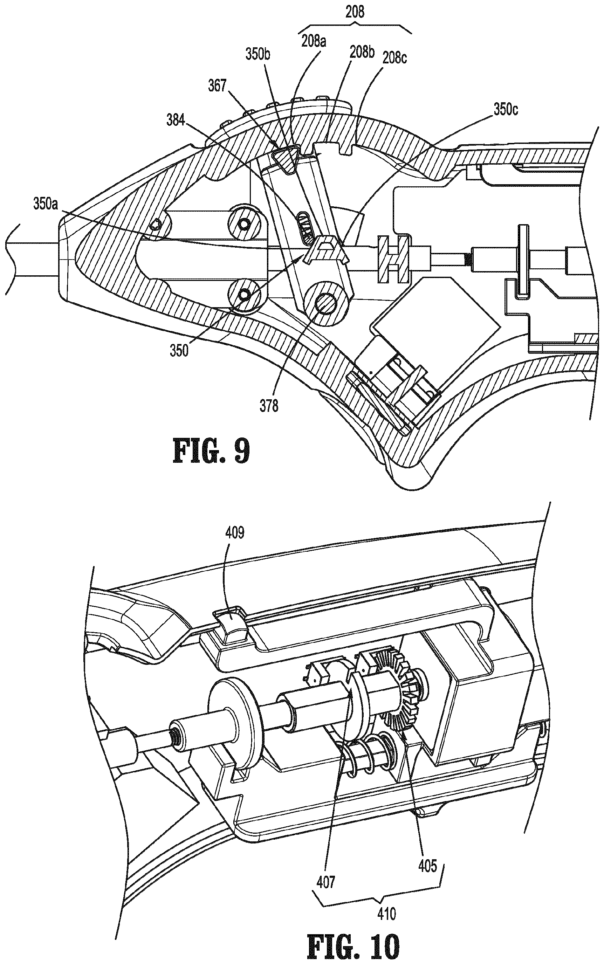

With reference now to FIG. 9 the articulation lever assembly 300 further includes a cam wedge 350 having first, second, and third portions 350a, 350b, 350c configured to cammingly engage the cam pin 384 which is cammingly slidable in the slots 374a, 374b of the articulation pivot arms 366a, 366b and the slot 363 of the articulation lever 360. The first, second, and third portions 350a, 350b, 350c correspond to the respective detent sections 208a, 208b, 208c of the detent portion 208. In this manner, articulation backlash is reduced as the cam pin 384 rides along the first, second, and third portions 350a, 350b, 350c of the cam wedge 350.

With reference back to FIGS. 6 and 7, the actuation assembly 400 includes an actuation rod 402 operatively coupled with the loading unit 30 (FIG. 2) of the surgical tack applier, a motor 420, an actuation switch 404 configured to actuate the motor 420 to eject the surgical tacks 10 (FIG. 4), a printed circuit board 430 including a microprocessor (not shown) to control the actuation assembly 400, and a battery pack 440 removably attached to the housing 202 and electrically connected to the motor 420 and the printed circuit board 430. A proximal end of the actuation rod 402 is operatively coupled with an output shaft of the motor 420 for concomitant rotation therewith such that when the actuation switch 404 is triggered by the clinician, the motor 420 is actuated to impart axial rotation to the actuation rod 402. A distal end of the actuation rod 402 is operatively coupled with the inner tube 38 (FIG. 3) of the loading unit 30 for concomitant rotation therewith.

With reference now to FIG. 10, the actuation assembly 400 may further include an encoder assembly 410 operatively connected to the actuation rod 402 and the processor of the printed circuit board 430. The encoder assembly 410 may include, e.g., an optical, motor encoder 405 configured to keep an accurate count of turns of the motor output shaft or the actuation rod 402 to ensure a proper number of turns are made to insert the surgical tack 10 through, e.g., the mesh "M", and into tissue "T" (FIG. 4). In addition, the encoder assembly 410 may further include, e.g., a single notched, encoder wheel 407 configured to ensure correct clocking of a distal end of the actuation rod 402 relative to the loading unit 30 (FIG. 2). The encoder assembly 410 may further include a light emitting diode ("LED") indicator 409 to indicate status of the ejection of each surgical tack 10. For example, a green light may indicate proper application of the surgical tack 10 through the mesh "M" and into tissue "T", and a red light may indicate, e.g., improper application of the surgical tack 10, due to an error signal from the optical motor encoder 405 or the single notched encoder wheel 407. Alternatively, the encoder assembly 410 may further include a piezoelectric element 411 (FIG. 6) for providing an audible tone for proper application of the surgical tack 10.

With brief reference to FIG. 6, the handle assembly 200 may further include a release lever 450 slidably attached to the housing 202. The release lever 450 is operatively coupled with the loading unit 30 (FIG. 2) such that when the release lever 450 is pulled, the loading unit 30 is detached from the elongate member 50 (FIG. 2) of the surgical tack applier.

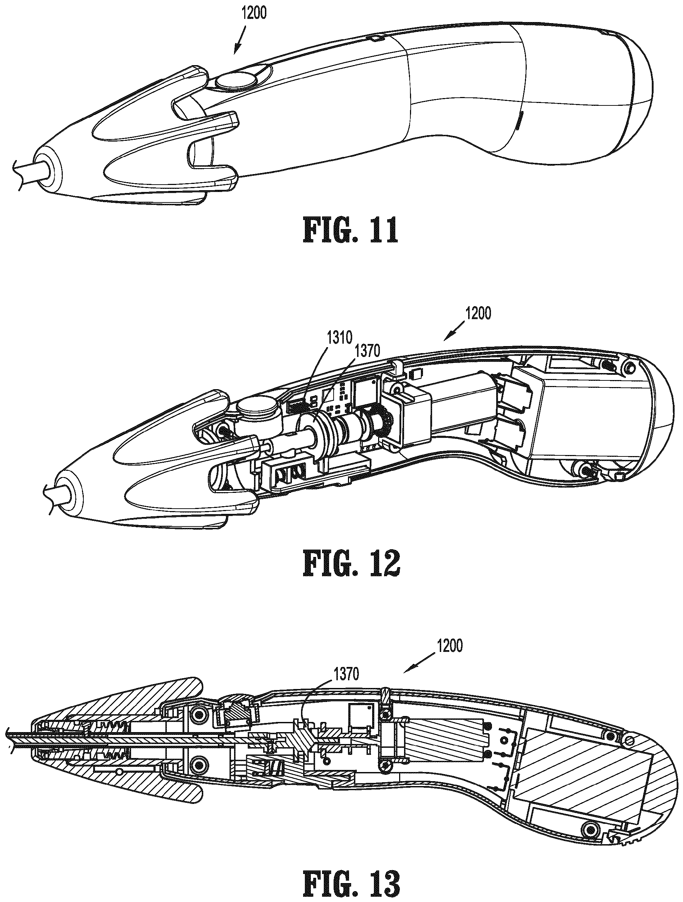

In use, the loading unit 30 is operatively mounted to a distal end of the elongate member 50. The loading unit 30 is introduced into a target surgical site while in the non-articulated condition. The clinician may remotely articulate loading unit 30 relative the longitudinal axis "X-X" to access the surgical site. Specifically, the clinician may slide the engaging portion 364 of the articulation lever 360 along the engaging surface 204 of the housing 202. As the articulation rod 310 is displaced axially, the loading unit 30 is moved to an articulated orientation relative to the central longitudinal axis "X-X". Furthermore, the clinician may position the surgical mesh "M" adjacent the surgical site. Once the surgical mesh "M" is properly positioned on the surgical site, the clinician may trigger the actuation switch 404 to eject a surgical tack 10 through the mesh "M" and into tissue "T". While the articulation rod 310 is configured for axial displacement, it is further contemplated that an actuation rod 1310 may be rotatably supported by a rotor 1370 such that the actuation rod 1310 outputs an axial rotation which may be utilized by the loading unit 30 to effect articulation thereof, as can be appreciated with reference to FIGS. 11-13. It is further contemplated that the actuation assembly 400 may further include a transmission assembly to selectively impart rotation of the output shaft of the motor 420 to the actuation rod 1310.

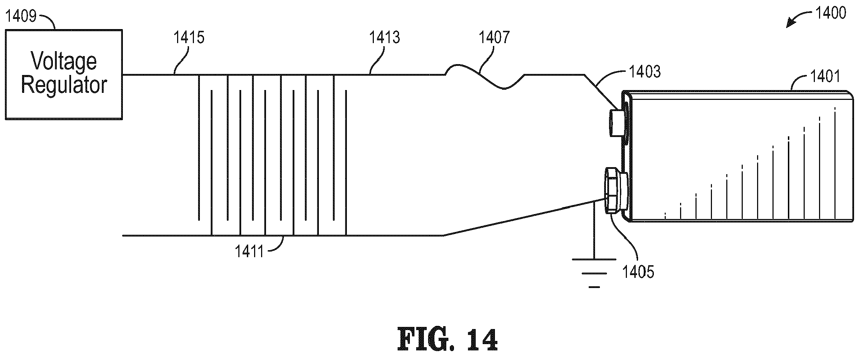

Aspects of safety cut-off circuits for use with powered surgical instruments such as the surgical tack applier described above are illustrated in FIGS. 14-16 and described in detail below. It is contemplated that the safety cut-off circuits of the disclosure are incorporated into the housing of the powered surgical instruments and may utilize a shared power supply with that of the powered surgical instrument or may include its own independent power supply. Although the safety cut-off circuits are described as including certain components, it is understood that aspects of the safety cut-off circuits may include some or all of the components described, as needed for any specific implementation. In aspects, the safety cut-off circuits described below are configured to electrically couple to other electrical components of the powered surgical instrument, and additionally or alternatively, may be controlled by microprocessors of the powered surgical instrument.

FIG. 14 illustrates a safety cut-off circuit 1400 which provides power cut-off when liquid is detected in the circuit to protect the electrical components of the surgical instrument. Safety cut-off circuit 1400 includes a power supply 1401, a fuse 1407, a liquid detection circuit 1411, and a voltage regulator 1409. The power supply 1401 includes a positive terminal 1403 and a negative terminal 1405 with the negative terminal 1405 being grounded, for example, to a chassis of a surgical instrument. The fuse 1407 is coupled in series to the positive terminal 1403 of the power supply 1401. The liquid detection circuit 1411 is coupled in parallel to the fuse 1407 and the negative terminal 1405 of the power supply 1401. The voltage regulator 1409 is operably coupled to the liquid detection circuit 1411 and the positive terminal 1403 of the power supply 1401 via the fuse 1407.

The fuse 1407 includes an amperage rating greater than an amperage rating required to operate the safety cut-off circuit 1400. The liquid detection circuit 1411 may include water detection traces and/or an interlaced comb structure.

During operation, power supplied to the voltage regulator 1409 is cut off when liquid comes into contact with the liquid detection circuit 1411. In particular, in the event of water or other liquid ingress into the surgical instrument that could corrupt the logic and signals of the microcontroller or other component of the surgical instrument, the power supply 1401 becomes short circuited due to detection of liquid by liquid detection circuit 1411. This creates a very high current draw in excess of what is required to operate the surgical instrument or circuit normally and causes fuse 1407 to blow. In aspects, first trace 1413, illustrated to the right of the liquid detection circuit 1411 in FIG. 14, is of a lower gauge value than second trace 1415, illustrated to the left of liquid detection circuit 1411 in FIG. 14, such that the first trace 1413 is large enough to handle the required current to blow the fuse 1407. As illustrated in FIG. 14, the liquid detection circuit 1411 is disposed upstream of any components required to establish logic on the board or in the surgical instrument. Disabling fuse 1407 cuts power to any microcontroller and all other circuitry components on the board or in the surgical instrument.

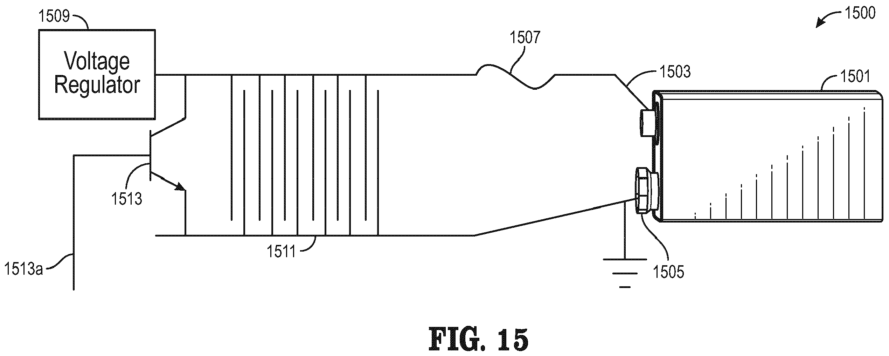

FIG. 15 illustrates a safety cut-off circuit 1500 similar to safety cut-off circuit 1400 and only the differences between the two will be described for brevity. While safety cut-off circuit 1400 is useful for preventing damage to the components of the surgical instrument when liquid is detected in the circuit, safety cut-off circuit 1500 is configured to cut-off the power supply 1501 upon the occurrence of other safety-related conditions.

Unlike safety cut-off circuit 1400, the liquid detection circuit 1511 of safety cut-off circuit 1500 is optional and may be removed from the circuit. Additionally, safety cut-off circuit 1500 includes a transistor 1513 coupled in parallel to the safety cut-off circuit 1500 which is configured to be selectively triggered to create a short circuit and blow the fuse 1507. The amperage capacity of the transistor 1513 is higher than an amperage capacity of the fuse 1507 to ensure that the fuse 1507 will blow before any damage is incurred on the transistor 1513 or any other components of the circuit and surgical instrument.

The transistor 1513 of the safety cut-off circuit 1500 includes a logic pin 1513a coupled to a microcontroller for selectively triggering the transistor 1513 to create the short circuit and blow the fuse 1507. The transistor 1513 is triggered to create the short circuit and blow the fuse 1507 when an end of useable life of the surgical instrument is detected, for example, for single use devices, upon completion of use of the surgical instrument. In an aspect, transistor 1513 is triggered to create the short circuit when liquid is detected elsewhere in the surgical instrument, not local to the liquid detection circuit 1511. Additionally or alternatively, when erroneous behavior or signals are detected from another component of the surgical instrument (e.g., another circuit in the surgical instrument, a motor, a power source, etc.), transistor 1513 may also be triggered by a microcontroller to blow fuse 1507.

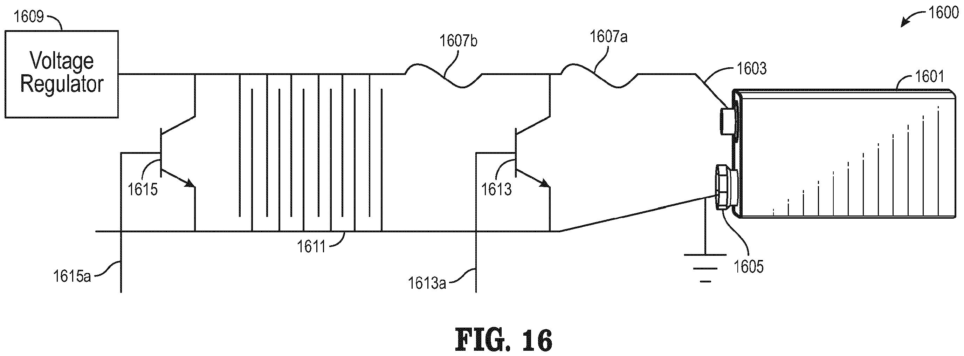

FIG. 16 illustrates another aspect of a safety cut-off circuit 1600 similar to safety cut-off circuit 1400 and safety cut-off circuit 1500 described above. Safety cut-off circuit 1600 provides power cut-off when liquid is detected in the circuit to protect the electrical components of the surgical instrument and also includes a first transistor 1613 and a second transistor 1615 for selectively cutting off portions of the circuit.

Safety cut-off circuit 1600 includes a power supply 1601, a fuse 1607a, a resettable fuse 1607b, a liquid detection circuit 1611, a first transistor 1613, a second transistor 1615, and a voltage regulator 1409. The power supply 1601 includes a positive terminal 1603 and a negative terminal 1605 with the negative terminal 1605 being grounded, for example, to a chassis of a surgical instrument. The fuse 1607a is coupled in series to the positive terminal 1603 of the power supply 1601 and the resettable fuse 1607b is coupled in series with the fuse 1607a. The first transistor 1613 is coupled in parallel, between the fuse 1607a and the resettable fuse 1607b. The liquid detection circuit 1611 is coupled in parallel to the resettable fuse 1607b and the negative terminal 1605 of the power supply 1601. The second transistor 1615 is coupled in parallel after the liquid detection circuit 1611. The voltage regulator 1609 is operably coupled to the liquid detection circuit 1611 and the positive terminal 1603 of the power supply 1601 via the fuse 1607a and the resettable fuse 1607b.

The liquid detection circuit 1411 may include water detection traces and/or an interlaced comb structure. The fuse 1607a and/or resettable fuse 1607b includes an amperage rating greater than an amperage rating required to operate the safety cut-off circuit 1600. Additionally, an amperage rating of the resettable fuse 1607b is less than an amperage rating of the fuse 1607a, such that the resettable fuse 1607b will blow before the fuse 1607a blows, or without the fuse 1607a blowing at all. Additionally, an amperage capacity of the first transistor 1613 is greater than an amperage capacity of the fuse 1607a and an amperage capacity of the second transistor 1615 is greater than an amperage capacity of the resettable fuse 1607b.

The first transistor 1613 includes a logic pin 1613a coupled to a microcontroller for selectively triggering the first transistor 1613 to create a short circuit and blow the fuse 1607a. Such an occurrence will permanently disable the safety cut-off circuit and protect the components of the surgical instrument. The second transistor 1615 includes a logic pin 1615a coupled to a microcontroller for selectively triggering the second transistor 1615 to create a short circuit and blow the resettable fuse 1607b. Such an occurrence of blowing the resettable fuse 1607b via the second transistor 1615 does not impact the fuse 1607a, and only temporarily disables the operation of the safety cut-off circuit 1600. Upon resetting the resettable fuse 1607b, the safety cut-off circuit 1600 functions normally.

During operation, power supplied to the voltage regulator 1609 is cut off when liquid comes into contact with the liquid detection circuit 1611, when first transistor 1613 is caused to blow fuse 1607a, or when second transistor 1615 is caused to blow resettable fuse 1607b. In particular, in the event of water or other liquid ingress into the surgical instrument that could corrupt the logic and signals of the microcontroller or other component of the surgical instrument, the power supply 1601 becomes short circuited due to detection of liquid by liquid detection circuit 1611. This creates a very high current draw in excess of what is required to operate the surgical instrument or circuit normally and causes fuse 1607a and/or resettable fuse 1607b to blow.

Safety cut-off circuit includes two additional components for disabling power. As described above, microcontroller logic can selectively trigger a transistor (e.g., first transistor 1613 or second transistor 1615) on the board that creates a short circuit to blow resettable fuse 1607b, to temporarily remove power, or blow fuse 1607a, to permanently remove power. The microcontroller could be programmed to do this for any number of reasons. The first transistor 1613 or second transistor 1615 can be triggered by microcontroller to create the short circuit and blow the fuse 1607a and/or the resettable fuse 1607b when an end of useable life of the surgical instrument is detected, for example, for single use devices, upon completion of use of the surgical instrument. In an aspect, the first transistor 1613 and/or the second transistor 1615 is triggered to create the short circuit when liquid is detected elsewhere in the surgical instrument, not local to the liquid detection circuit 1611. Additionally or alternatively, when erroneous behavior or signals are detected from another component of the surgical instrument (e.g., another circuit in the surgical instrument, a motor, a power source, etc.), the first transistor 1613 may also be triggered by a microcontroller to blow fuse 1607a and/or the second transistor 1615 may be triggered by a microcontroller to blow resettable fuse 1607b.

Persons skilled in the art will understand that the structures and methods specifically described herein and shown in the accompanying figures are non-limiting exemplary aspects, and that the description, disclosure, and figures should be construed merely as exemplary of particular aspects. It is to be understood, therefore, that the disclosure is not limited to the precise aspects described, and that various other changes and modifications may be effected by one skilled in the art without departing from the scope or spirit of the disclosure.

Additionally, the elements and features shown or described in connection with certain aspects may be combined with the elements and features of certain other aspects without departing from the scope of the disclosure, and that such modifications and variations are also included within the scope of the disclosure. Accordingly, the subject matter of the disclosure is not limited by what has been particularly shown and described.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A safety cut-off circuit for a surgical instrument, the safety cut-off circuit comprising:
   a power supply including a positive terminal and a negative terminal, the negative terminal being grounded;
   a fuse coupled in series to the positive terminal of the power supply;
   a liquid detection circuit coupled in parallel to the fuse and the negative terminal of the power supply; and
   a voltage regulator operably coupled to the liquid detection circuit and the positive terminal of the power supply via the fuse, wherein power supplied to the voltage regulator is cut-off when liquid comes into contact with the liquid detection circuit.
2. The safety cut-off circuit of paragraph 1, wherein the fuse is configured to blow when liquid contacts the liquid detection circuit.
3. The safety cut-off circuit of paragraph 1, wherein the liquid detection circuit is coupled to the fuse via a first trace and to the voltage regulator via a second trace and the first trace is of a lower gauge relative to the second trace.
4. The safety cut-off circuit of paragraph 1, wherein the liquid detection circuit includes water detection traces.
5. The safety cut-off circuit of paragraph 1, wherein the liquid detection circuit includes an interlaced comb structure.
6. The safety cut-off circuit of paragraph 1, wherein the fuse includes an amperage rating greater than an amperage rating required to operate the safety cut-off circuit.
7. The safety cut-off circuit of paragraph 1, further comprising a transistor coupled in parallel to the safety cut-off circuit and configured to be selectively triggered to create a short circuit and blow the fuse.
8. The safety cut-off circuit of paragraph 7, wherein an amperage capacity of the transistor is higher than an amperage capacity of the fuse.
9. The safety cut-off circuit of paragraph 7, wherein the transistor includes a logic pin coupled to a microcontroller for selectively triggering the transistor to create the short circuit and blow the fuse.
10. The safety cut-off circuit of paragraph 7, wherein the transistor is triggered to create the short circuit and blow the fuse when at least one of an end of useable life is detected, liquid is detected elsewhere in the surgical instrument remote from the liquid detection circuit, or erroneous behavior or signals are detected from at least one other electrical component of the surgical instrument.
11. The safety cut-off circuit of paragraph 10, wherein the at least one other electrical component of the surgical instrument includes a motor or a power source.
12. The safety cut-off circuit of paragraph 1, further comprising a resettable fuse coupled in series to the fuse, wherein an amperage rating of the resettable fuse is less than an amperage rating of the fuse.
13. The safety cut-off circuit of paragraph 12, further comprising a first transistor and a second transistor, wherein an amperage capacity of the first transistor is greater than an amperage capacity of the fuse and an amperage capacity of the second transistor is greater than an amperage capacity of the resettable fuse.
14. The safety cut-off circuit of paragraph 13, wherein the second transistor includes a logic pin coupled to a microcontroller for selectively triggering the second transistor to create a short circuit and blow the resettable fuse.
15. A safety cut-off circuit for a surgical instrument, the safety cut-off circuit comprising:
   a power supply including a positive terminal and a negative terminal, the negative terminal being grounded;
   a fuse coupled in series to the positive terminal of the power supply;
   at least one of a liquid detection circuit or a transistor coupled in parallel to the fuse and the negative terminal of the power supply; and
   a voltage regulator operably coupled to at least one of the liquid detection circuit or the transistor and the positive terminal of the power supply via the fuse, wherein power supplied to the voltage regulator is cut-off when at least one of liquid comes into contact with the liquid detection circuit or the transistor is caused to short circuit the safety cut-off circuit and blow the fuse.
16. A powered surgical instrument comprising:
   a handle assembly comprising:
      an actuation assembly including a motor;
      an actuation rod having a first end operatively coupled to an output shaft of the motor for concomitant rotation therewith; and
      an actuation switch configured to actuate the motor;
   an articulation lever assembly including an articulation rod and an articulation lever operatively coupled with the articulation rod;
   an elongate member extending distally from the handle assembly and including a loading unit having a plurality of surgical tacks; and
   a safety cut-off circuit operably coupled to at least one of the handle assembly, the articulation lever assembly, or the elongate member, the safety cut-off circuit comprising:
      a power supply including a positive terminal and a negative terminal, the negative terminal being grounded;
      a fuse coupled in series to the positive terminal of the power supply;
      a liquid detection circuit coupled in parallel to the fuse and the negative terminal of the power supply; and
      a voltage regulator operably coupled to the liquid detection circuit and the positive terminal of the power supply via the fuse, wherein power supplied to the voltage regulator is cut-off when liquid comes into contact with the liquid detection circuit.

## Claims

1. A safety cut-off circuit for a surgical instrument, the safety cut-off circuit comprising:
a power supply including a positive terminal and a negative terminal, the negative terminal being grounded;
a fuse coupled in series to the positive terminal of the power supply;
a liquid detection circuit coupled in parallel to the fuse and the negative terminal of the power supply; and
a voltage regulator operably coupled to the liquid detection circuit and the positive terminal of the power supply via the fuse, wherein power supplied to the voltage regulator is cut-off when liquid comes into contact with the liquid detection circuit.

2. The safety cut-off circuit of claim 1, wherein the fuse is configured to blow when liquid contacts the liquid detection circuit.

3. The safety cut-off circuit of claim 1 or claim 2, wherein the liquid detection circuit is coupled to the fuse via a first trace and to the voltage regulator via a second trace and the first trace is of a lower gauge relative to the second trace.

4. The safety cut-off circuit of any preceding claim, wherein the liquid detection circuit includes water detection traces.

5. The safety cut-off circuit of any preceding claim, wherein the liquid detection circuit includes an interlaced comb structure.

6. The safety cut-off circuit of any preceding claim, wherein the fuse includes an amperage rating greater than an amperage rating required to operate the safety cut-off circuit.

7. The safety cut-off circuit of any preceding claim, further comprising a transistor coupled in parallel to the safety cut-off circuit and configured to be selectively triggered to create a short circuit and blow the fuse.

8. The safety cut-off circuit of claim 7, wherein an amperage capacity of the transistor is higher than an amperage capacity of the fuse.

9. The safety cut-off circuit of claim 7, wherein the transistor includes a logic pin coupled to a microcontroller for selectively triggering the transistor to create the short circuit and blow the fuse.

10. The safety cut-off circuit of claim 7, wherein the transistor is triggered to create the short circuit and blow the fuse when at least one of an end of useable life is detected, liquid is detected elsewhere in the surgical instrument remote from the liquid detection circuit, or erroneous behavior or signals are detected from at least one other electrical component of the surgical instrument; preferably wherein the at least one other electrical component of the surgical instrument includes a motor or a power source.

11. The safety cut-off circuit of any preceding claim, further comprising a resettable fuse coupled in series to the fuse, wherein an amperage rating of the resettable fuse is less than an amperage rating of the fuse.

12. The safety cut-off circuit of claim 11, further comprising a first transistor and a second transistor, wherein an amperage capacity of the first transistor is greater than an amperage capacity of the fuse and an amperage capacity of the second transistor is greater than an amperage capacity of the resettable fuse.

13. The safety cut-off circuit of claim 12, wherein the second transistor includes a logic pin coupled to a microcontroller for selectively triggering the second transistor to create a short circuit and blow the resettable fuse.

14. A safety cut-off circuit for a surgical instrument, the safety cut-off circuit comprising:
a power supply including a positive terminal and a negative terminal, the negative terminal being grounded;
a fuse coupled in series to the positive terminal of the power supply;
at least one of a liquid detection circuit or a transistor coupled in parallel to the fuse and the negative terminal of the power supply; and
a voltage regulator operably coupled to at least one of the liquid detection circuit or the transistor and the positive terminal of the power supply via the fuse, wherein power supplied to the voltage regulator is cut-off when at least one of liquid comes into contact with the liquid detection circuit or the transistor is caused to short circuit the safety cut-off circuit and blow the fuse.

15. A powered surgical instrument comprising:
a handle assembly comprising:
an actuation assembly including a motor;
an actuation rod having a first end operatively coupled to an output shaft of the motor for concomitant rotation therewith; and
an actuation switch configured to actuate the motor;
an articulation lever assembly including an articulation rod and an articulation lever operatively coupled with the articulation rod;
an elongate member extending distally from the handle assembly and including a loading unit having a plurality of surgical tacks; and
a safety cut-off circuit operably coupled to at least one of the handle assembly, the articulation lever assembly, or the elongate member, the safety cut-off circuit comprising:
a power supply including a positive terminal and a negative terminal, the negative terminal being grounded;
a fuse coupled in series to the positive terminal of the power supply;
a liquid detection circuit coupled in parallel to the fuse and the negative terminal of the power supply; and
a voltage regulator operably coupled to the liquid detection circuit and the positive terminal of the power supply via the fuse, wherein power supplied to the voltage regulator is cut-off when liquid comes into contact with the liquid detection circuit.
